# EUROPEAN PATENT APPLICATION

(11) **EP 3 804 696 A1**
(43) Date of publication of application: **14.04.2021**
(21) Application number: 20200504.7
(22) Date of filing: 07.10.2020
(51) Int. Cl.: A61K 9/00, A61K 47/22, A61K 31/19, A61K 31/22, A61K 31/34, A61K 31/60, A61K 31/704, A61K 31/728, A61K 36/489, A61K 36/53, A61K 36/61, A61K 36/82, A61K 36/896, A61P 17/12

(54) **A MULTI-COMPONENT WART DESTRUCTING MEANS**

(30) Priority: 07.10.2019 DK PA201970625
(71) Applicant: Unigroup ApS, 2800 Lyngby (DK)
(72) Inventor: Licht, Flemming, 2800 Kongens Lyngby (DK)
(74) Representative: Holme Patent A/S

(57) **Abstract**

A multi-component wart destructing means (4,9) comprises a first component (4) adapted for topical application of at least one first compound (7) onto a wart (3), and a separate second component (9) independent of the first component (4), which second component (9) is adapted for application onto the wart (3) to invade the wart (3) after topical application of the at least one first compound (7).

## Description

The present invention relates to a multi-component wart destructing means, and to a method for destructing warts.

Traditional means for destructing warts include amongst other cryotherapy, application of silver nitrate, wart remover gel keratolytics, and recently also application of bleomycin by means of micro needle patches or by needle injections directly into the wart.

Cryotherapy is usually performed by application of liquid nitrogen to the wart. Cryotherapy needs to be done by skilled staff every 2 or 3 weeks for several months before the wart is fully removed. Moreover the application of liquid nitrogen is difficult to keep within the border of the wart and healthy tissue is also freezed, thereby making a much larger lesion than the area of the wart.

Silver nitrate is corrosive and destroys skin cells. The corrosive properties of this ingredient make it very useful in removing affected skin tissue. Silver nitrate works by destroying very thin layers of tissue at a time, both pathological and non-pathological. As the layer of skin dies, the next layer must then be applied to ensure penetration of the wart tissue continues. It takes time and requires skin to be smooth. Loose skin must be removed prior to the silver nitrate application. Silver nitrate is however toxic, corrosive, very poisonous and can cause burns, so handling of silver nitrate should not be done carelessly. Long-term exposure can also cause permanent blue-grey staining of skin, and short contact can lead to deposition of black silver stains on the skin. Such silver nitrate discolorations are very disfiguring and difficult, or even impossible to get rid of.

Wart remover gel keratolytics may be based on topically applied salicylic acid that helps cause the wart to gradually peel off as with silver nitrates and freezing. However side effects, such as spreading skin redness around the treated area, signs of infection, e.g. pus, milky or bloody discharge, and formation of a deep sore at the treatment site, are side effects.

Bleomycin is known as a chemotherapy drug. The study Efficacy of Intralesional Bleomycin in Palmo-plantar and Periungual Warts, J Cutan Aesthet Surg. 2011 Sep-Dec; 4(3): 188-191 concluded that intralesional injection of bleomycin is highly effective, safe, and non-toxic in periungual and palmo-plantar warts. Intralesional injection of bleomycin can thus be used to treat viral warts that have failed to respond to the above conventional therapies but must be used under guidance by health staff.

International patent application no. WO2017/204418 relates to microneedle patches containing bleomycin for wart treatment. This known microneedle array comprises a pad base and a plurality of microneedles protruding from the pad base and a drug coating comprising bleomycin. The microneedle array is made of biodegradable material that slowly degrades to simultaneous and gradually release the bleomycin to the wart onto which the microneedle patch is applied. Thus the microneedle patches contains the active wart destructing pharmaceutical medicament. But due to being a one-component system the bleomycin is applied to a substantial area of healthy tissue next to the wart tissue. Furthermore Bleomycin is an anti-cancer chemotherapy drug, and like all chemotherapy drugs, bleomycin can cause serious side effects.

The applicant's own European patent application no. EP2460509A1 concerns a one-component system including a composition for topical treatment of warts in humans. The composition comprises formic acid and at least one C1 - C4 alkyl ester of lactic acid, malic acid, tartaric acid, citric acid, or a mixture of any of these. Preferably said at least one C1 - C4 alkyl ester is ethyl lactate. The advantage of this known composition is that hard skin covering the warts need not be removed prior to the topical application.

The known means and methods of the prior art to destruct warts suffer from several disadvantage, as mentioned above, including the discoloration, lack of control of local application of the treatment to the wart tissue without lesioning the surrounding healthy skin, high toxicity, being costly, etc.

Therefore it is a main aspect of the present invention to provide a gentle means and method that can be used and carried out, respectively, locally, topically and substantially exclusively to the wart with minimum side effects and injury to the tissue around the wart.

It is a further aspect of the present invention to provide a bleomycin-free means to destruct warts.

It is a further aspect of the present invention to provide a means to destruct warts that are simple to use and can be used for self-treatment.

It is a further aspect of the present invention to provide a means to destruct warts that does not involve a health hazard to the patient.

It is a further aspect of the present invention to provide a means to destruct warts that is effective inside the wart and not only from the skin surface.

The novel and unique features whereby these and other aspects are achieved according to the present inventions consist in that the multi-component wart destructing means comprises
- a first component adapted for topical application of at least one first compound onto a wart, and
- a separate second component independent of the first component, which second component is adapted for application onto the wart to invade the wart after topical application of the at least one first compound.

Within the context of the present application and invention the term "invade" or "invading" is understood to mean the ability to penetrate into tissue to affect injuriously and progressively whether it being mechanically, chemically or both.

Within the context of the present application and invention the term "wart" or "warts" includes any kind of warts including the five main types of warts: common warts (aka vurruca vulgaris), plantar warts (aka plantar warts), flat warts, filiform warts, and genital warts. The present invention is applicable to any kind of mammal warts, in particular human warts.

Warts are caused by infection with a type of human papillomavirus (HPV), and the HPV virus is believed to enter the body through skin that has been damaged slightly. The virus triggers extra cell growth, which makes the outer layer of skin thick and hard. Warts can grow anywhere on the skin, but the most frequent warts are on hands or feet. The warts are very disfiguring and may be embarrassing, e.g. if present in high numbers or being large and protruding. Warts on the bottom of the feet may be very painful. Warts are highly contagious and are mainly passed by direct skin contact or spread with things like towels or razors that have touched a wart, so warts should be treated.

Within the context of the present application and invention the term "topical" or "topical medication" means that a component or a compound is applied epicutaneous, thus directly to a specific spot on the skin of the human body, in the present case to the wart.

Within the context of the present application and invention the term "multi-component" means that the wart destructing means is composed of two or more components, but not necessarily two or more chemical compounds. The combined action of the first component and the second component provides the wart destruction in a way that is gentler and less irritating to the skin area around the wart than hitherto known when using known wart destruction means. The onset of the destructive action of the multi-component wart destructing means of the present invention is triggered by the application of the second component after application of the first component, which triggering may be mechanical or chemical. The preferred multi-component system is a two-component system in which both components have an active role.

The conventional systems for destructing warts are based on the action of a single component whether it be injection of bleomycin directly into the wart, freezing of the wart, etching gels, or similar. Although a plaster may be applied subsequently to the treated wart such a plaster does not take part in the destructive action, but is only for protection purpose to avoid spreading of virus and cover the wart.

In a preferred embodiment of the present invention the second component may be a microneedle patch. The microneedle patch confers a plurality of micro-lesions in the area of the microneedles, which micro-lesions activate the immune system and cause fluid from the surrounding tissue to be attracted to said micro lesions, whereby the at least one first compound that has been applied to the wart exterior surface in advance by the first component is drawn inside the wart tissue with the result that wart tissue is attacked from both inside the wart and from the skin surface thereby speeding up wart destruction time substantially. The tissue around the wart is not affected because no or just an inferior amount of at least one first compound is applied by accident or flowing outside the physical border of the wart, and because the microneedles only can draw the at least one first compound inside the wart at the area treated with said at least one first compound, not outside said area. Thus, even if the area of microneedles is larger than the area of the wart having an application of the at least one first compound the destructive action is kept at the wart area where the at least one first compound is applied. Moreover the at least one first compound is drawn very fast inside the wart, within seconds, leaving little time for spreading of the at least one first compound to surrounding tissue.

The microneedle patch may be of any conventional kind, or be specifically produced for the present invention. Optionally the microneedle patch may comprise at least one second compound, which second compound may comprise one or more compound(s) selected from the group comprising a second organic acid, ethyl lactate, at least one extract from a plant part, and an antibacterial agent, and combinations thereof. The least one second compound may react with the at least one first compound when the microneedle patch is placed on the wart, including reacting with the at least one first compound inside the wart and on the wart surface, to substantially increase reaction surface area for wart destruction. The at least one second compound may also contribute to attracting fluid, such as water, to the reaction site. Thus the at least one second compound substantially increases penetration speed of the at least one first compound down into the wart tissue.

Although the injuries and lesions caused by the microneedles may act to increase the contagiousness of the wart the microneedle patch at the same time encloses the treated wart to prevent spreading of HPV. No additional plasters are needed.

Advantageously the at least one second compound may comprise a compound selected from the group comprising one or more of a second organic acid, an ester, at least one extract from a plant part, and an antibacterial agent, and combinations thereof.

The at least one second compound may take part in a chemical reaction with the at least one first compound, e.g. cleavage of the at least one first compound, and as a result lowering pH inside the wart tissue. The at least one second compound may achieve a synergistic effect with the at least one first compound.

The second organic acid may advantageously works by increasing the amount of moisture in the skin and dissolving the substances that causes the skin cells to stick together. This makes it easier to shed the wart cells after the at least one first component, or a derivate of the at least one first component, has initially acted on the wart tissue.

The second organic acid may e.g. be a C1 - C14 organic acid, preferably caprylic acid and/or hyaluronic acid, and/or the ester be a second ester, preferably ethyl lactate.

Hyaluronic acid is one of the components of connective, epithelial, and neural tissues, forming a gelatinous matrix that surrounds cells. Hyaluronic acid is a natural component of the human body and takes part in the development and architecture of tissues and organs. Through oxygen radicals or via hyaluronidase, glucuronidase and hexosaminidase in sites of tissue injury, intact hyaluronic acid may be depolymerized into hyaluronic acid fragments, which are able to activate the innate immune defense and promote the production of different cytokines (Saari and Konttinen 1989; Gariboldi et al. 2008). The microneedles provide a plurality of paths inside the wart for the hyaluronic acid to activate the innate immune defense.

Hyaluronic acid is thus a humectant that keeps the skin hydrated by attracting water from within the dermis and moving the water along to the epidermis. Hyaluronic acid can also attract water from other surrounding environment, such as the air. So a further beneficial effect of the hyaluronic acid is that it attracts further water to the wart environment to activate the first component.

Caprylic acid is an oily liquid that is minimally soluble in water and has antibacterial and antifungal properties. Caprylic acid is known to e.g. accelerate β-oxidation for the formation of acetyl-coA akin, and to increase the rate of astrocyte ketogenesis. The advantageous effect of caprylic acid in the present invention is believed to be partly due to its oily consistency in combination with the acidic properties (pKa 4.89).

The at least one second compound may have a large molecular size to effectively be simply absorbed into the skin and needs a vehicle, such as the microneedle patch, for transportation inside the wart tissue along with the at least one first compound.

Ethyl lactate will, in the presence of water and acids (or bases), hydrolyze into lactic acid and ethanol, wherein in particular lactic acid (pKa 3.86) has wart destructing properties. Accordingly, when the at least one second compound is ethyl lactate, it is preferred that the at least one first compound comprises an organic acid and/or water, thereby providing a simple and effective multi-component wart destructing means according to the invention.

Water is however not a mandatory compound of the first component in that water and humid are inherently attracted to the application site upon application of any first compound. For example are tricholoroacetic acid and formic acid hygroscopic, and the attraction of humid, moisture and/or water is further enhanced when the second compound is added, in particular when the second component is a microneedle patch containing hyaluronic acid.

Water as a solvent for any of the first compound or second compound can be replaced with an organic solvent suited for said compound, thus not deteriorating the valuable chemical and physical properties of said compounds. The solvent can e.g. be a physiological acceptable alcohol or oil.

Many extracts from plant parts are known to have a beneficial effect corresponding to, replacing, and/or adding to the effects of the at least one second compound. As examples can be mentioned one or more of antibacterial, anti-inflammatory, antiviral, and antifungal properties.

The at least one second compound may include various kinds of plant extracts or other compounds that have one of more of the above properties, serve as adjuvants or being suspension vehicles for the ingredients of the at least one second compound.

Such additional second compounds can e.g. be Origanum vulgare leaf extract, marine algae, Kigelia africana fruit extract, Piper nigrum, Borago Officinalis Seed oil, Rosmarinus Officinalis Leaf Extract, Mel, Vitis vinifera extract, Sambucus nigra extract, Melaleuca alternifolia, salicylic acid, green tea extract, madecassoside, Totarol and Allicin, aloe vera extract, castor oil, Camellia sinensis extract, furyl compounds, such as furocoumarins and furanochromones, alkaloids, such as β-carbolines, furanoquinolines, camptothecin, atropine, caffeine, indolizidines swainsonine, castanospermine, colchicines, and vinblastine, polyacetylenes (polyines), polysaccharides, and thiophenes.

Other examples of at least one extract from a plant part include but are not limited to plant extracts selected from the group comprising Sophora Angustifolia root extract, Asiaticoside, Paris polyphylla yannensis extract, Camellia sinensise polyphenols, Totarol, EGCG (Epigallocatechin gallate), Madecassoside, Vitamin C, retinal peptide, EGCE (Green tea extract), Collagen, Adenosine, Arbutin, Niacin amide, and Glycyrrhiza. In the alternative the active ingredient of the at least one extract from plant parts can be obtained using a method other than extracting said ingredient from a plant part. Alternative methods may include any known chemical, physical, and biological method.

Some extracts from plant parts do however induce a discoloration. If for example formic acid is mixed with ethyl lactate, organic vulgare leaf extract and caprylic acid this formulation is unstable and turns into a disfiguring brownish solution over time. This disadvantage is remedied by the multi-component wart destructing means of the present invention where the compounds that might cause discolorations are kept apart during storage.

The at least one first compound to be transported inside the wart tissue by the second component, with or without inclusion of the at least one second compound, may comprise a compound selected from the group comprising one or more of a first organic acid, an ester, an inorganic acid, dimethyl isosorbide, and water, and combinations thereof.

In some embodiment of the multi-component wart destructing means of the present invention the first organic acid may comprise a C1-C8 organic acid, and combinations thereof.
In a preferred embodiment the first organic acid may be formic acid, acetic acid, and/or trichloroacetic acid.

In another preferred embodiment the at least one first compound is an ester in form of a first ester, preferably ethyl lactate, wherein the at least one second compound may advantageously initiate hydrolysis of the first ester so that the acid is set free only after hydrolysis to act to destruct wart tissue.

In this embodiment with a first ester of e.g. a C1 - C8 organic acid, the release and action of the carboxylic acid is controlled to onset upon contact of the at least one first compound with the at least one second compound, thus mainly inside and on top of the wart. The tissue around the wart is not affected because contact between the at least one first compound and the at least one second compound is limited to the wart tissue.

The at least one first compound topically applied to the wart can however be any kind of acid, such as formic acid, lactic acid or trichloroacetic acid, e.g. in combination with water. The at least one first compound can in some embodiments even be water.

The at least one first compound can also be ethyl lactate that hydrolyzes upon reaction with the at least one second compound to release wart destructing lactic acid in a hydrous environment, preferably wherein the at least one second component includes or is caprylic acid.

A person skilled in the art will, based on the present application, understands that the first compound may be the second compound and vice versa.

In a preferred embodiment of the present invention the micro needle patch is enriched with a second compound in form of a composition of caprylic acid, e.g. 0.9 wt/wt%, and Origanum vulgare leaf extract, e.g. 0.1 wt/wt%, and one of formic acid, lactic acid or ethyl lactate, or combinations thereof, be the first compound(s) topically applied directly to the wart before the micro needle patch is place on top of the wart.
However in the alternative the micro needle patch could include the formic acid, lactic acid, or ethyl lactate, or combinations thereof, as the second compound, and the composition of caprylic acid and Origanum vulgare leaf extract be applied topically to the wart.

The microneedle patch may have a plurality of microneedles, wherein the microneedles have one or more of the features of
- containing the least one second compound, or
- having an exterior coating of the least one second, or
- being biodegradable, or
- being made of a material that dissolves when applied to the wart.

The central part of the patch base may also have a coating or a content of the at least one second compound.

Advantageous features of a microneedle patch for use in the present invention includes but are not limited to the features listed below:
- the volume of the at least one second compound in the micro needle patch is sufficient to provide a wart reducing and/or destructing effect,
- the number of microneedles in the microneedle patch may be between 1 - 900,
- the microneedle length may be between 200 - 800µm, preferably between 450 - 500µm,
- a microneedle penetration depth into the wart of between 200 - 800µm,
- at least the patch base is made of hydrocolloid, or another similar material,
- a penetration time of the at least one second component from the microneedles into cutis of approximately 1 second or longer,
- a dissolving time of the at least one second component from the microneedles into cutis of approximately 1 second - 2 hours, or longer.

A person skilled in the art will understand that a sufficient volume of the second compound for providing the desired wart reducing/destruction effect depends on the second compound used. For instance, when the second compound is a plant extract, the volume may e.g. be between 1 - 5 wt/wt% of the total weight of the micro needle patch, and when the second compound is a ethyl lactate the volume may be between 1 and 80 wt/wt% of the total weight of the micro needle patch, e.g. between 5 wt/wt% or 20 and 60 w/w%.

To provide optimal control of the application of the first component the first component may be a dispenser having an applicator tip adapted for topical administration to the wart of the at least one first compound, which at least one first compound may be accommodated in a compound reservoir in fluid communication with the applicator tip to deliver the at least one first compound onto the wart exterior surface.

Advantageously the dispenser may be configured as for example a pen, a jet injector, a syringe, or similar, which configurations are known in the art to the patient or user, and therefore particular easy to use and manipulate for proper control of application of the at least one first compound to the wart exterior surface. Preferably the dispenser in non-invasive to the wart, thus without injection needles. For example the applicator tip may be configured to dispense a drop of the at least one first compound or be a pad adapted to dap the at least one first compound onto the wart exterior surface.

The present invention further relates to using the wart destructing means in the topical destruction of warts.

The use comprises the steps of
a) providing the wart destructing means described above,
b) applying the at least one first compound topically to the exterior surface of the wart,
c) applying the second component to the wart on top of the at least one first compound, and
d) leaving the second component at, on and/or inside the wart during an initial period, preferably the initial period is at least 1 second, preferably between 10 seconds and 24 hours, and in case the second component is a microneedle patch containing at least one second compound the initial period is at least the period corresponding to completion of delivery of the at least one second compound to the wart. The microneedle patch can, but need not, be replaced with other kind of protection, such as a conventional plaster or dressing.

The method may be seen as a cosmetic method.

In one embodiment the second component in form of a microneedle patch may be applied to the wart for a short time less than 24 hours, since typical release time of the at least one second component from the microneedles or dissolving time of the microneedles is about 2 hours where after the microneedle patch can be removed. However, in a different embodiment the microneedle patch may be left on the wart for a longer period e.g. up to one week, or even longer if preferred, thereby both protecting said ward and preventing the wart from contaminating the surroundings.

If a first approach does not suffice to destruct the wart a second and any further approach can be done without the tissue around the wart suffers from unintentional irritation and lesions as in the known treatments. To do that the method may, but need not, further comprise a step e) of replacing the exhausted second component of step d) with a new second component and repeating steps b) to d).

However continued use should be as short as possible, for some warts less than a week, up to maximum 12 weeks.

Various non-limiting compositions of compounds of the wart destructing means within the scope of the present invention are given below.

### Example 1

First compounds: 16 grams of crystalline trichloroacetic acid is added to a flask and ethyl lactate (60 wt/wt%) is added up to 40 ml.

Second compound: None added to the hyaluronic acid of the microneedle patch.

Ethyl lactate is a monobasic ester, and will, in the presence of water and acids hydrolyze into the wart destruction component lactic acid in the dermis and/or epidermis.

The combination of formic acid and ethyl lactate to remove corn and callus is known within the art of treatment. When replacing formic acid with trichloroacetic acid it is possible to have a much higher concentration of ethyl lactate in the at least one first component. As an example: in a 85 wt/wt% formic acid formulation only up to 15 wt/wt% ethyl lactate could be added, whereas 40 wt/wt% ethyl lactate is possible when combined with 60 wt/wt% trichloroacetic acid. Tests performed by the inventor confirm that the penetration into cutis of the combination of trichloroacetic acid and ethyl lactate is much higher compared to trichloroacetic acid and water, and without the sensation of pain, blistering and burning sensation.

Thus, in one embodiment relatively high concentrations of trichloroacetic acid, i.e. above 40w/v% is combined with ethyl lactate in order to provide the first compound. In said embodiment water is preferably the second compound e.g. applied via the micro needle patch.

Alternatively, the first compound may comprises (or consist of) trichloroacetic acid and water, and the second compound comprises (or consist of) ethyl lactate. In said embodiment the concentration of trichloroacetic acid is preferably around 40 wt/wt%.

Alternatively, the first compound may be water.

The flow in a dispenser in form of a pen applicator is considerably. Thus more first compound(s) can be applied in controlled manner to the wart. The more volume that can be applied to the wart the better and faster effect. Yet an advantage is, as mentioned above, that trichloroacetic acid is extreme hygroscopic attracting humidity, thereby causing efficient hydrolysis of the ester, the ethyl lactate. So when adding ethyl lactate instead of water, the concentration of trichloroacetic acid can also be higher without side effects compared to a simple aqueous solution of trichloroacetic acid. The preferred concentration ranges are 35-65 w/v% trichloroacetic acid and 35-65 wt/wt% ethyl lactate.

### Example 2

First compounds: Formic acid 85 wt/wt% + water 15 wt/wt%. Second compound: None added to the hyaluronic acid of the microneedle patch.

No ester amongst the first compounds means that no hydrolysis takes place. The first compounds are carboxylic acids mechanically transported by the microneedles in the micro lesions made by said microneedles while the hyaluronic acid attracts water. Once the transport is completed the microneedles may dissolve or not.

### Example 3

First compounds: Formic acid 85 wt/wt% + Dimethyl isosorbide 15 wt/wt%.

Second compound: None added to the hyaluronic acid of the microneedle patch.

No ester amongst the first compounds means that no hydrolysis takes place. Actions as in Example 2.

### Example 4

First compounds: Formic acid 80 wt/wt% or Trichloroacetic acid 50 w/wt%, the rest is ethyl lactate.

Second compounds in the microneedle patch: Water (5 wt/wt%); Hyaluronic acid (55 wt/wt%), Sophora Angustifolia (9 wt/wt%), Asiaticoside (5 wt/wt%), Paris Polyphylla yunnanensis (6 wt/wt%), Melaleuca alternifolia (tea tree) leaf oil (4 wt/wt%), Camellia sinensis polyphenols (5 wt/wt%), and salicylic acid (11 wt/wt%).

Most preferred plant extracts in the at least one second component include Origanum vulgare leaf extract, Paris Polyphylla yunnanensis, Camellia sinensis polyphenols, and Sophora Angustifolia.

### Example 5

First compounds: 85 wt/wt% formic acid and 15 wt/wt% dimethyl isosorbide.

Second compounds added to the hyaluronic acid of the microneedle patch: 0.1 wt/wt% origanum vulgare leaf extract and 0.9 wt/wt% caprylic acid, the rest being water (and/or inert excipients/fillers). Optionally, one or more further second compounds may be added.

### Example 6

First compounds: 85 wt/wt% formic acid or lactic acid.

Second compounds added to the hyaluronic acid of the microneedle patch: 0.1 wt/wt% Origanum vulgare leaf extract, 0.9 wt/wt% caprylic acid, and 10 wt/wt% ethyl lactate, the rest being water (and/or inert excipients/fillers). Optionally, one or more further second compounds may be added.

It is normally not possible to place a plaster on top of a wart treated with amongst others formic acid, as this will lead to soreness in the wart. However, when the second compound is added to the wart (after application of the first compound) using a microneedle patch, the inventors of the present invention has found out that said patch can be placed on the wart for a prolonged period of time, e.g. more than a week, without initiating any soreness. Without being bound by theory, this is likely due to the fact that the microneedle patch provides a plurality of micro-lesions in the wart, such that the at least one first and/or the at least one second compound is/are drawn inside the wart whereby the wart tissue is attacked from both inside the wart and from the skin surface.

The invention will now be briefly described in principle by reference to the drawing in which
Fig. 1 shows a hand with a wart on a finger, and application of first compound(s) on the wart,
Fig. 2 is an enlarged scale perspective view of a microneedle patch, and
Fig. 3 shows the microneedle patch applied to the wart after application of the first compound.

The wart is by way of example a finger wart, but warts of any kind and on any tissue can be treated with the multi-component wart destructing means of the present invention.

The microneedle patch is shown to be circular with a plurality of projecting microneedles. Emphasis is made that the size of the microneedle patch, its dimension in the relation between surrounding area without microneedles and centre area with micro needles, the number, shape and size of the microneedle are shown for illustrative purposes and modifications and variations are within the scope of the present invention.

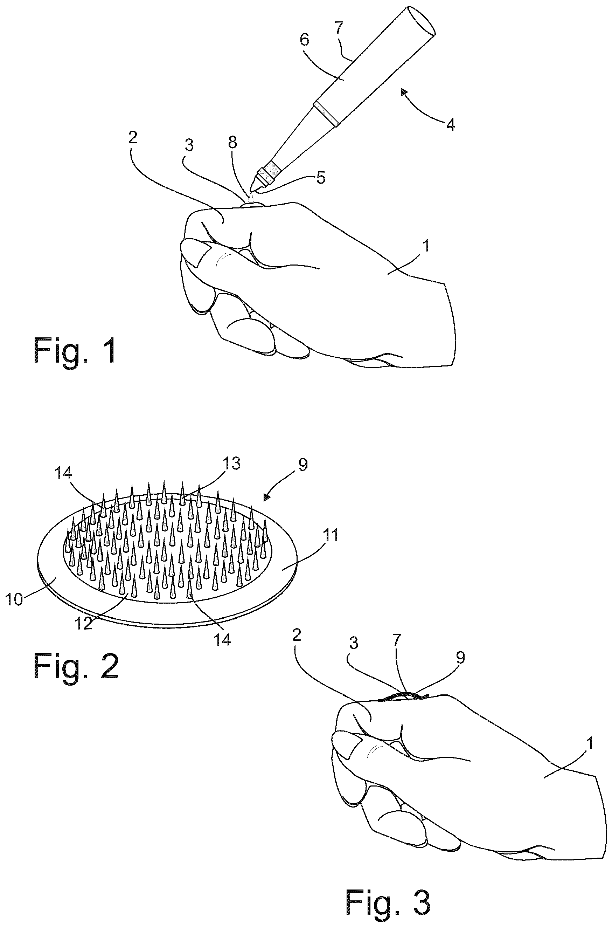

Fig. 1 shows a hand 1 having an index finger 2 with a wart 3. A pen applicator 4 has an applicator tip 5 and a reservoir 6 of first compound(s) 7, a drop 8 of which is being applied to the wart 3.

The microneedle patch 9 shown in fig. 2 has a patch base 10 that has a rim zone 11 free of micro needles and a central zone 12 delimited by the rim zone 11 and having a plurality of projecting microneedles 13 to be inserted into the wart 3. The rim zone 11 may be adhesive to fasten the microneedle patch 9 around the wart 3. At least the pad base 10 may be made of hydrocolloid. The microneedles may be made of dehydrated, solid hyaluronic acid.

The microneedle patch 9 is adapted to be applied on top of the wart 3, as seen in fig. 3, to allow a coating of second compound(s) 14, or a volume of second compound(s) 14 inside the microneedles 13, or both a coating and a volume of second compound (s) to be released from the microneedles 13 of the microneedle patch 9 to the warts tissue below the free skin surface. The release can take place by either the microneedles 13 that have a content of the second compound(s) 14 gradually dissolve by contract by the attracted water, or by the coating of the second compound (s) 14 on the microneedles 13 being released during contact with water attracted by the lesion made by the microneedles and/or water attracted by the second compound(s) 14.

## Claims

1. A multi-component wart destructing means (4,9) **characterised in** comprising
- a first component (4) adapted for topical application of at least one first compound (7) onto a wart (3), and
- a separate second component (9) independent of the first component (4), which second component (9) is adapted for application onto the wart (3) to invade the wart after topical application of the at least one first compound (7).

2. A multi-component wart destructing means (4,9) according to claim 1, **characterised in that** the second component (9) is a microneedle patch (9).

3. A multi-component wart destructing means (4,9) according to claim 2, **characterised in that** the microneedle patch (9) comprises at least one second compound (14).

4. A multi-component wart destructing means according to claim 3, **characterised in that** the at least one second compound comprises one or more compound (s) selected from the group comprising a second organic acid, an ester, at least one extract from a plant part, and an antibacterial agent, and combinations thereof.

5. A multi-component wart destructing means according to claim 4, **characterised in that**
- the second organic acid is a C1 - C14 organic acid, preferably caprylic acid and/or hyaluronic acid, and/or
- the ester is a second ester in form of ethyl lactate.

6. A multi-component wart destructing means (4,9) according to any of the preceding claims 1 - 5, **characterised in that** the at least one first compound comprises one or more compound(s) selected from the group comprising a first organic acid, a first ester, an inorganic acid, dimethyl isosorbide, and water, and combinations thereof.

7. A multi-component wart destructing means (4,9) according to claim 6, **characterised in that** the first organic acid comprises a C1-C8 organic acid, and/or combinations thereof.

8. A multi-component wart destructing means (4,9) according to any of claims 6 - 7, **characterised in that** the first organic acid is formic acid, acetic acid, and/or trichloroacetic acid.

9. A multi-component wart destructing means (4,9) according to any of the preceding claims 2 - 8, **characterised in that** the microneedle patch (9) has a plurality of microneedles (13), wherein the microneedles (13) have one or more of the features of
- containing the least one second compound (14), or
- having an exterior coating of the least one second compound (14), or
- being biodegradable,
- being made of a material that dissolve when applied to the wart (3).

10. A multi-component wart destructing means (4,9) according to any of the preceding claims 1 - 9, **characterised in that** the first component (7) is a dispenser (4) having an applicator tip (5) adapted for topical administration to the wart (3) of the at least one first compound (7), which at least one first compound (7) is accommodated in a compound reservoir (6) in fluid communication with the applicator tip (5).

11. A multi-component wart destructing means (4,9) according to any of the preceding claims 4 - 10, **characterised in that** the at least one extract from a plant part is selected from the group comprising Origanum vulgare leaf extract, marine algae, Kigelia africana fruit extract, Piper nigrum, Borago Officinalis Seed oil, Rosmarinus Officinalis Leaf Extract, Mel, Vitis vinifera extract, Sambucus nigra extract, Melaleuca alternifolia, salicylic acid, green tea extract, madecassoside, Totarol, Allicin, aloe vera extract, castor oil, Camellia sinensis extract, furyl compounds, such as furocoumarins and furanochromones, alkaloids, such as β-carbolines, furanoquinolines, camptothecin, atropine, caffeine, indolizidines swainsonine, castanospermine, colchicines, and vinblastine, polyacetylenes (polyines), polysaccharides, and thiophenesSophora Angustifolia root extract, Asiaticoside, Paris polyphylla yannensis extract, Camellia sinensise polyphenols, Totarol, EGCG, Madecassoside, Vitamin C, retinal peptide, EGF, Collagen, Adenosine, Arbutin, Niacin amide, and Glycyrrhizat and combinations thereof.

12. A multi-component wart destructing means (4,9) according to any of the preceding claims 1 - 10 for use in topical treatment of wart, **characterised in that** the topical treatment comprises providing the multi-component wart destructing means (4,9) according to any of the preceding claims 1 - 11,
**a)** applying the at least one first compound (7) topically to the exterior surface of the wart (3),
**b)** applying the second component (14) to the wart (3), and
**c)** leaving the second component (14) at, on and/or inside the wart (3) during an initial period, preferably the initial period is at least 1 second, preferably between 10 seconds and 24 hours, and in case the second component is a microneedle patch (9) containing at least one second compound (14), wherein the initial period is at least the period corresponding to completion of delivery of the at least one second compound (14) to the wart (3).
